(19) Europäisches Patentamt
European Patent Office
Office européen des brevets

(11) EP 1 922 320 B1

(12) # FASCICULE DE BREVET EUROPEEN

(45) Date de publication et mention de la délivrance du brevet:
**27.10.2010 Bulletin 2010/43**

(21) Numéro de dépôt: **06794297.9**

(22) Date de dépôt: **07.08.2006**

(51) Int Cl.:
***C07D 471/18*** *(2006.01)* ***A61K 31/439*** *(2006.01)*
***A61P 25/00*** *(2006.01)*

(86) Numéro de dépôt international:
**PCT/FR2006/001911**

(87) Numéro de publication internationale:
**WO 2007/020343 (22.02.2007 Gazette 2007/08)**

(54) **DERIVES DE 5-PYRIDAZINYL-1-AZABICYCLO[3.2.1]OCTANE, LEUR PREPARATION ET LEUR APPLICATION EN THERAPEUTIQUE**

5-PYRIDAZINYL-1-AZABIZYCLO[3.2.1]OCTAN-DERIVAT, HERSTELLUNGSVERFAHREN UND DEREN VERWENDUNG FÜR THERAPEUTIKA

DERIVATIVES OF 5-PYRIDAZINYL-1-AZABICYCLO[3.2.1]OCTANE, PREPARATION METHOD THEREOF AND USE OF SAME IN THERAPEUTICS

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **18.08.2005 FR 0508594**

(43) Date de publication de la demande:
**21.05.2008 Bulletin 2008/21**

(73) Titulaire: **Sanofi-Aventis**
**75013 Paris Cedex (FR)**

(72) Inventeurs:
- **GALLI, Frédéric**
  **F-92420 Vaucresson (FR)**
- **LECLERC, Odile**
  **F-91300 Massy (FR)**
- **LOCHEAD, Alistair, W.**
  **F-94220 Charenton (FR)**
- **VACHE, Julien**
  **F-75011 Paris (FR)**

(74) Mandataire: **Gaslonde, Aude et al**
**Sanofi-Aventis**
**Département Brevets**
**174, Avenue de France**
**75013 Paris (FR)**

(56) Documents cités:
**WO-A-00/34279** **WO-A-00/34284**
**WO-A-03/057697**

- **HOLLADAY ET AL: "Neuronal Nicotinic Acetylcholine Receptros as Targets for Drug Discovery" 19 décembre 1997 (1997-12-19), JOURNAL OF MEDICINAL CHEMISTRY, AMERICAN CHEMICAL SOCIETY. WASHINGTON, US, PAGE(S) 4169-4194 , XP002124847 ISSN: 0022-2623 page 4180; figure 5; exemples 43-54**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 922 320 B1

## Description

La présente invention se rapporte à des dérivés de 5-pyridazinyl-1-azabicyclo[3.2.1]octane, à leur préparation et à leur application en thérapeutique.

La présente invention a pour objet les composés répondant à la formule générale (I)

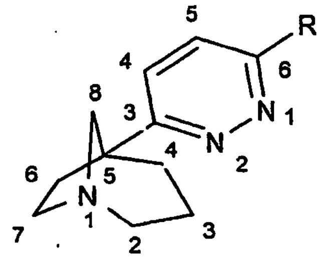

(I)

dans laquelle :

R représente
soit un atome d'hydrogène ou d'halogène ;
soit un groupe phényle éventuellement substitué par un ou plusieurs atomes d'halogène, par un ou plusieurs groupes choisis parmi les groupes $(C_1-C_5)$alkyle, $(C_1-C_6)$alcoxy, nitro, amino, di$(C_1-C_3)$alkylamino, trifluorométhyle, trifluorométhoxy, cyano, hydroxy, acétyle ou méthylènedioxy ;
soit un groupe choisi parmi un pyridinyle, pyrazolyle, imidazolyle, triazolyle, tétrazolyle, oxazolyle, thiazolyle, oxadiazolyle, thiadiazolyle, thiényle, furyle, isoxazolyle, isothiazolyle, pyrrolyle, naphtyle, ce groupe pouvant éventuellement être substitué par un ou plusieurs groupes choisis parmi les atomes d'halogènes, les groupes $(C_1-C_6)$alkyle, $(C_1-C_6)$alcoxy, trifluorométhoxy, trifluorométhyle, nitro, cyano, hydroxy, amino, $(C_1-C_6)$alkylamino ou di$(C_1-C_6)$alkylamino.

Par ailleurs, l'atome de carbone en position 5 du cycle azabicyclo[3.2.1]octane est asymétrique, de sorte que les composés de l'invention peuvent exister sous forme de deux énantiomères ou de mélange de ces derniers. Ces énantiomères ainsi que leurs mélanges, y compris les mélanges racémiques, font partie de l'invention.

Les composés de formule (I) peuvent également exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :

- un atome d'halogène : un atome de fluor, de chlore, de brome ou d'iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe alcoxy : un radical -O-alkyle dont le groupe alkyle est tel que précédemment défini.

Parmi les composés de formule (I) objets de l'invention, un premier sous-groupe de composés est constitué par les composés pour lesquels :

R représente
soit un atome d'halogène, plus particulièrement un chlore ;
soit un groupe phényle éventuellement substitué par un ou plusieurs atomes d'halogène, plus particulièrement de chlore ou de fluor, par un ou plusieurs groupes choisis parmi les groupes $(C_1-C_6)$alkyle, plus particulièrement méthyle, $(C_1-C_6)$alcoxy, plus particulièrement méthoxy ;

soit un groupe choisi parmi un pyridinyle, pyrazolyle, imidazolyle, triazolyle, tétrazolyle, oxazolyle, thiazolyle, oxadiazolyle, thiadiazolyle, thiényle, furyle, isoxazolyle, isothiazolyle, pyrrolyle, naphtyle, ce groupe pouvant éventuellement être substitué par un ou plusieurs groupes ($C_1$-$C_6$)alkyle, plus particulièrement méthyle.

Parmi les composés de formule (I) objets de l'invention, un second sous-groupe de composés est constitué par les composés pour lesquels :

R représente
soit un atome d'halogène, plus particulièrement un chlore ;
soit un groupe phényle éventuellement substitué par un ou plusieurs atomes d'halogène, plus particulièrement de chlore ou de fluor, par un ou plusieurs groupes choisis parmi les groupes ($C_1$-$C_6$)alkyle, plus particulièrement méthyle, ($C_1$-$C_6$)alcoxy, plus particulièrement méthoxy ;
soit un groupe choisi parmi un pyridinyle, pyrazolyle, imidazolyle, thiényle, furyle, pyrrolyle, ce groupe pouvant éventuellement être substitué par un ou plusieurs groupes ($C_1$-$C_6$)alkyle, plus particulièrement méthyle.

Dans ce qui suit, on entend par groupe protecteur un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316.

Conformément à l'invention, on peut préparer les composés de formule générale (I) par un procédé illustré par le schéma 1 qui suit.

On fait réagir le composé de formule (II), dans laquelle $PPh_3$ désigne un groupe triphénylphosphane, avec le glyoxylate d'éthyle pour obtenir un composé de formule (III). La réduction de la double liaison éthylénique fournit un composé de formule (IV) qu'on fait réagir avec l'hydrate d'hydrazine pour obtenir un composé de formule (V). On traite ce dernier par du brome dans l'acide acétique pour obtenir un composé de formule (VI). Le traitement de ce composé par de l'oxychlorure de phosphore conduit au composé de formule (VII). Les composés de formule (I) peuvent ensuite être préparés à partir du composé de formule (VII) selon toutes méthodes connues, telles que par exemple :

- avec un acide boronique de formule $R\text{-}B(OH)_2$ dans laquelle R est tel que défini dans la formule générale (I), en présence d'un catalyseur au palladium, par exemple le tétrakistriphénylphosphine palladium ;
- avec un composé de formule R-H dans laquelle R est tel que défini dans la formule générale (I), en présence d'une base forte, par exemple l'hydrure de sodium, dans un solvant, par exemple le diméthylformamide ;
- avec un dérivé stanneux de formule $R\text{-}Sn[(CH_2)_3CH_3)]_3$ dans laquelle R est tel que défini dans la formule générale (I), en présence d'un catalyseur au palladium, par exemple le bis(triphénylphosphino)dichloropalladium ;
- avec un composé de formule R-H dans laquelle R est tel que défini dans la formule générale (I), en présence de n-butyllithium, de chlorure de zinc et d'un catalyseur au palladium, par exemple le tétrakistriphénylphosphine palladium.

## Schéma 1

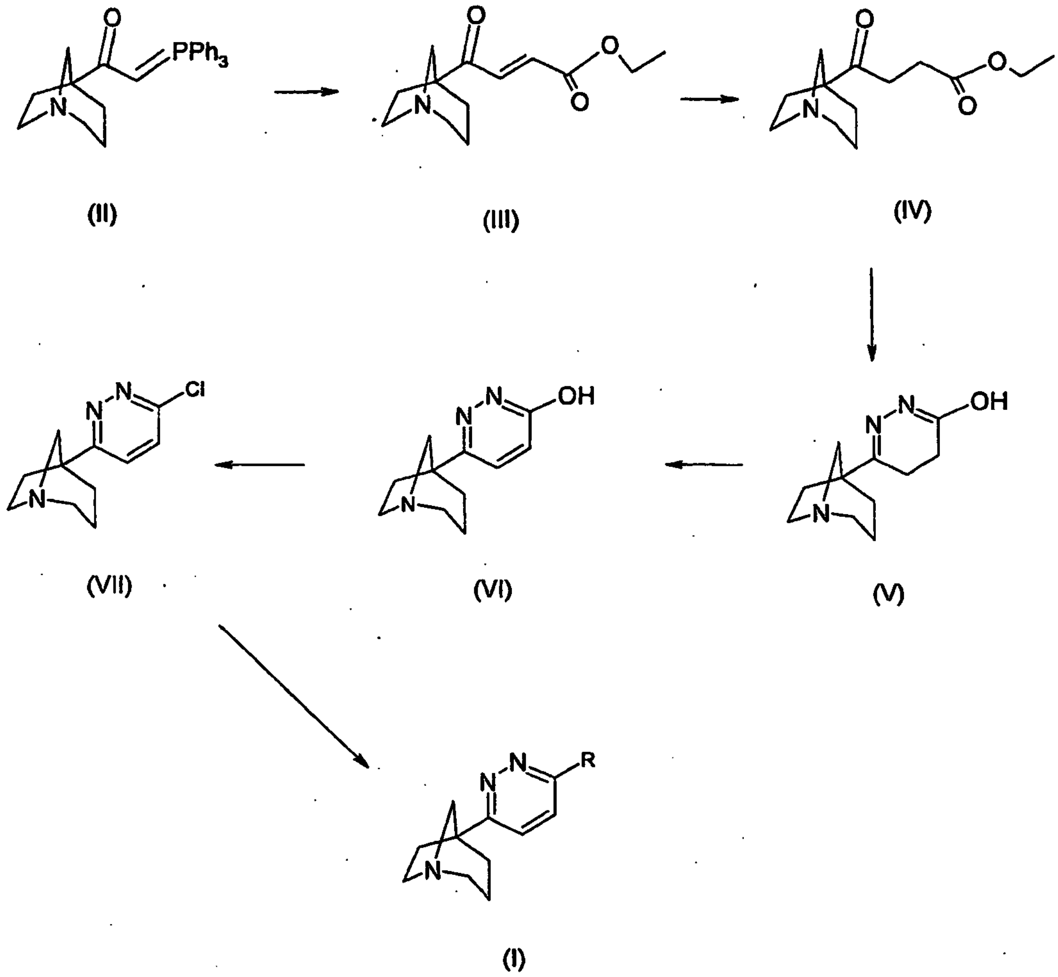

(II) (III) (IV)

(VII) (VI) (V)

(I)

Le composé de formule (II) est accessible par des méthodes décrites dans la littérature, comme par exemple dans J. Med. Chem. 1992, 2392.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

L'invention, selon un autre de ses aspects, a également pour objet les composés de formules (II) à (VII). Ces composés sont utiles comme intermédiaires de synthèse des composés de formule générale (I).

Les exemples suivants décrivent la préparation de certains composés conformes à l'invention. Ces exemples ne sont pas limitatifs et ne font qu'illustrer la présente invention. Les numéros des composés donnés entre parenthèses dans les titres renvoient à ceux donnés dans la première colonne du tableau ci-après, qui illustre les structures chimiques et les propriétés physiques de quelques composés selon l'invention.

**<u>Exemple 1</u> (composé N°1)**

5-(6-phénylpyridazin-3-yl)-1-azabicyclo[3.2.1]octane

1.1. (2*E*)-4-(1-azabicyclo[3.2.1]oct-5-yl)-4-oxobut-2-énoate d'éthyle Dans un ballon ballon tricol de 100 ml on introduit 5,00 g (12,09 mmoles) de 1-(1-azabicyclo[3.2.1]oct-5-yol)-2-(triphénylphosphanylidène)éthanone en solution dans 20 ml de chloroforme et 20 ml de toluène. On ajoute 1,36 g (13,3 mmoles) de glyoxylate d'éthyle et on agite le milieu à température ambiante pendant 15 min. On élimine le solvant par évaporation sous pression réduite et on purifie

le résidu obtenu par chromatographie sur colonne de gel de silice en éluant par un mélange de chloroforme, méthanol et ammoniaque dans les proportions 89/210,2.

On obtient 1,44 g de produit sous forme de solide amorphe.

1.2. 4-(1-azabicyclo[3.2.1]oct-5-yl)-4-oxobutanoate d'éthyle

Dans un flacon d'hydrogénation on introduit 2,95 g (12,43 mmoles) de (2*E*)-4-(1-azabicyclo[3.2.1]oct-5-yl)-4-oxobut-2-énoate d'éthyle, tel que obtenu à l'étape 1.1, en solution dans 100 ml d'alcool éthylique, en présence de 0,4 g de palladium à 5% adsorbé sur charbon. On agite le milieu sous environ 0,28 MPa d'hydrogène pendant 1 h à température ambiante puis on le filtre sur terre de diatomées et on élimine le solvant par évaporation sous pression réduite.

On obtient 2,97 g de produit attendu sous forme de solide amorphe.

1.3. 6-(1-azabicyclo[3.2.1]oct-5-yl)-4,5-dihydropyridazin-3-ol

Dans un ballon tricol de 250 ml on introduit 2,90 g (12,12 mmoles) de 4-(1-azabicyclo[3.2.1]oct-5-yl)-4-oxobutanoate d'éthyle, obtenu à l'étape 1.2, en solution dans 50 ml d'alcool éthylique. On ajoute ensuite 1,94 g (60,59 mmoles) d'hydrate d'hydrazine et on chauffe le milieu réactionnel au reflux pendant 15 h. On évapore le solvant à sec sous pression réduite et on purifie le résidu par chromatographie sur colonne de gel de silice en éluant par un mélange de chloroforme, méthanol et ammoniaque dans les proportions 90/10/1.

On obtient 1,6 g de produit attendu sous forme de solide amorphe.

1.4. Bromhydrate (1 : 1) de 6-(1-azabicyclo[3.2.1]oct-5-yl)pyridazin-3-ol

Dans un ballon tricol de 50 ml on introduit 1,54 g (7,43 mmoles) de 6-(1-azabicyclo[3.2.1]oct-5-yl)-4,5-dihydropyridazin-3-ol, obtenu à l'étape 1.3, en solution dans 20 ml d'acide acétique. On chauffe le milieu à 70°C et on ajoute 1,31 g (8,17 mmoles) de brome. On agite pendant 15 min et on ajoute à nouveau 1,31. g (8,17 mmoles) de brome. On chauffe ensuite le milieu réactionnel à 100°C pendant 2 h. On élimine le solvant par filtration et on triture le résidu dans le méthanol. On collecte les cristaux obtenus par filtration sous pression réduite. On obtient 2 g de produit attendu.

Point de fusion : 196-198°C

1.5. 5-(6-chloropyridazin-3-yl)-azabicyclo[3.2.1]octane

Dans un ballon tricol de 50 ml on introduit 2,1 g (7,34 mmoles) de bromhydrate (1:1) de 6-(1-azabicydo[3.2.1]oct-5-yl)pyridazin-3-ol, tel que obtenu à l'étape 1.4, en solution dans 15 ml d'oxychlorure de phosphore. On chauffe le milieu réactionnel à 130°C pendant 30 min puis on le verse sur 500 ml d'eau glacée. On alcalinise ensuite la phase aqueuse par addition d'une solution aqueuse à 30% d'hydroxyde de sodium et on l'extrait par du chloroforme. On sèche les phases organiques réunies sur sulfate de magnésium, on les filtre et on les concentre sous pression réduite.

On obtient 1,56 g de produit sous forme de solide.

Point de fusion : 139-141°C

1.6. (+) ou (-)-5-(6-chloropyridazin-3-yl)-1-azabicyclo[3.2.1]octane

Le mélange racémique du 5-(6-chloropyridazin-3-yl)-1-azabicyclo[3.2.1]octane, obtenu à l'étape 1.5, est dédoublé par chromatographie liquide sur support chiral de façon à obtenir les énantiomères dextrogyre et lévogyre, respectivement, le (+)-5-(6-chloropyridazin-3-yl)-1-azabicyclo[3.2.1]octane et le (-)-5-(6-chloropyridazin-3-yl)-1-azabicyclo[3.2.1]octane.

(+)-5-(6-chloropyridazin-3-yl)-1-azabicyclo[3.2.1]octane : $[\alpha_D^{20}] = +30{,}9°$ (c = 1, $CH_3OH$)

(-)-5-(6-chloropyridazin-3-yl)-1-azabicyclo[3.2.1]octane : $[\alpha_D^{20}] = -17°$ (c = 1, $CH_3OH$)

1.7. 5-(6-phénylpyridazin-3-yl)-1-azabicyclo[3.2.1]octane

Dans un ballon tricol de 25 ml on introduit successivement 0,3 g (1,34 mmole) de 5-(6-chloropyridazin-3-yl)-1-azabicyclo[3.2.1]octane, obtenu à l'étape 1.5, et 0,245 g (2,01 mmoles) d'acide phénylboronique en solution dans 8 ml de toluène. On ajoute ensuite 1,42 ml (2,84 mmoles) d'une solution aqueuse 2M de carbonate de sodium, 1,5 ml d'éthanol et 0,0465 g (0,04 mmole) de tétrakis(triphénylphosphino)palladium. On chauffe le mélange au reflux pendant

20 h, on le refroidit à température ambiante, et on le verse sur 20 ml d'eau. On extrait la phase aqueuse trois fois avec 30 ml de chloroforme, on sèche les phases organiques réunies sur sulfate de magnésium et on les concentre sous pression réduite. On purifie le résidu par chromatographie sur colonne de gel de silice en éluant par un mélange chloroforme, méthanol et ammoniaque dans les proportions 95/5/0,5.

On obtient ainsi 0,32 g de produit attendu sous forme de cristaux.

Point de fusion : 133-134°C.

RMN $^1H$ ($CDCl_3$) δ (ppm): 8.15 (2H, d); 7.85 (1H, d); 7.60-7.40 (4H, m); 3.40-2.80 (6H, m) ; 2.30 (2H, t) ; 2.15-1.85 (2H, m) ; 1.80 (1H, s) ; 1.70-1.50 (1H, m).

Les composés n°2, 3 et 14 ont été préparés selon la méthode décrite dans l'exemple 1.

**Exemple 2 (compose N°5)**

Chlorhydrate (1 : 1) de 5-[6-(5-méthyl-2-thiényl)pyridazin-3-yl]-1-azabicyclo[3.2.1]octane

On obtient ce composé selon la méthode décrite à l'étape 1.6 de l'exemple 1, à partir de 5-(6-chloropyridazin-3-yl)-1-azabicyclo[3.2.1]octane, préparé selon l'étape 1.5 de l'exemple 1, et d'acide 5-méthylsulfanyl-2-thiénylboronique. On en prépare le chlorhydrate par traitement de la base par une solution d'acide chlorhydrique dans le propan-2-ol et on collecte les cristaux obtenus par filtration et séchage sous vide.

Point de fusion : 245-246°C.

RMN $^1H$ (DMSO) δ (ppm): 8.15 (1H, d); 7.75 (1H, d); 7.75 (1H, d); 6.95 (1H, d); 3.75-3.25 (6H, m) ; 2.45 (3H, s) ; 2.15-1.85 (6H, m).

Les composés n°6 à 11 ont été préparés selon la méthode décrite dans l'exemple 2.

Les composés n°12, 13, 16, 17, 22 et 23 ont été préparés selon la méthode décrite dans l'exemple 2, à partir de (+)-5-(6-chloropyridazin-3-yl)-1-azabicyclo[3.2.1]octane, obtenu par dédoublement du mélange racémique (préparé à l'étape 1.5 de l'exemple 1) par chromatographie liquide sur support chiral.

Les composés n°15, 20 et 21 ont été préparés selon la méthode décrite dans l'exemple 2, à partir de (-)-5-(6-chloropyridazin-3-yl)-azabicyclo[3.2.1]octane, obtenu par dédoublement du mélange racémique (préparé à l'étape 1.5 de l'exemple 1) par chromatographie liquide sur support chiral.

**Exemple 3 (composé N°25)**

(+)-5-[6-(-1*H*-imidazol-1-yl)pyridazin-3-yl]-1-azabicyclo[3.2.1]octane

Dans un ballon tricol de 10 ml on introduit 0,228 g (3,35, mmoles) d'imidazole en solution dans 4 ml de diméthylformamide. On ajoute ensuite 0,137 g (3,42 mmoles) d'hydrure de sodium en dispersion à 60% dans l'huile et on agite à température ambiante pendant 1 heure. Le mélange est alors additionné à une solution de (+)-5-(6-chloro-pyridazin-3-yl)-1-azabicyclo[3.2.1]octane (obtenu par dédoublement du mélange racémique préparé à l'étape 1.5 de l'exemple 1 par chromatographie liquide sur support chiral) (0,15 g, 0,67 mmole) dans la diméthylformamide et le milieu réactionnel est chauffé à 90°C pendant 15 heures puis à 110°C pendant 3 heures et le solvant est évaporé sous pression réduite. Le résidu est repris dans 10 ml de chloroforme et 10 ml d'une solution aqueuse saturée de carbonate de sodium. La phase aqueuse est extraite à nouveau par 10 ml de chloroforme et les phases organiques réunies sont lavées par une solution aqueuse saturée de chlorure de sodium, séchées sur sulfate de sodium, filtrées et concentrées sous pression réduite. Le résidu est purifié par chromatographie sur plaque de gel de silice en éluant par un mélange de chloroforme, méthanol et ammoniaque dans les proportions 85/15/1,5. On obtient 0,111 g de produit attendu.

Point de fusion : 177-179°C

RMN $^1H$ (DMSO) δ (ppm): 8.55 (1H, s); 8.10 (1H, d); 8.00 (1H, s); 7.85 (1H, d); 7.15 (1H; s); 3.15-2.70 (6H, m); 2.25-1.75 (5H, m); 1.60-1.35 (1H, t).

Le composé n°26 a été préparé selon la méthode décrite dans l'exemple 3.

**Exemple 4 (composé N°19)**

Chlorhydrate (2:1) de (+)-5-[6-(-1*H*-imidazol-4-yl)pyridazin-3-yl]-1-azabicyclo[3.2.1]-octane

4.1. (+)-5-[6-(-1-triphénylméthyl-imidazol-4-yl)pyridazin-3-yl]-1-azabicyclo[3.2.1]octane Dans un ballon tricol de 10 ml on introduit successivement 0,14 g (0,63 mmole) de (+)-5-(6-chloropyridazin-3-yl)-1-azabicyclo[3.2.1]octane (obtenu par dédoublement du mélange racémique préparé à l'étape 1.5 de l'exemple 1 par chromatographie liquide sur support chiral) (0,15 g, 0,67 mmole) en solution dans 3 ml de tétrahydrofurane, 0,94g .(1,56 mmole) de 1-triphénylméthyl-4-tributylstannyl imidazole et 0,027 g (0,037 mmole) de bis(triphénylphosphino)dichloropalladium. Le mélange est alors

chauffé à 100°C sous atmosphère d'argon pendant 15 heures puis dilué dans 10 ml de chloroforme et 10 ml d'une solution aqueuse saturée de carbonate de sodium. La phase aqueuse est extraite à nouveau par 10 ml de chloroforme et les phases organiques réunies sont lavées par une solution aqueuse saturée de chlorure de sodium, séchées sur sulfate de sodium, filtrées et concentrées sous pression réduite. Le résidu est purifié par chromatographie sur colonne de gel de silice en éluant par un mélange de chloroforme, méthanol et ammoniaque dans les proportions 96/4/0,4. On obtient le produit attendu, contaminé par l'excès de 1-triphénylméthyl-4-tributylstannyl imidazole, sous forme de solide amorphe.

4.2. Chlorhydrate (2:1) de (+)-5-[6-(-1*H*-imidazol-4-yl)pyridazin-3-yl]-1-azabicyclo-[3.2.1]octane

Dans un ballon tricol de 10 ml on introduit le résidu de (+)-5-[6-(-1-triphénylméthylimidazol-4-yl)pyridazin-3-yl]-1-azabicyclo[3.2.1]octane, obtenu à l'étape 4.1, en solution dans 4 ml de méthanol. On ajoute ensuite 0,6 ml d'une solution d'acide chlorhydrique 6N dans l'alcool isopropylique et on chauffe le milieu réactionnel à 80°C pendant 3 heures. Le solvant est concentré sous pression réduite et le résidu est trituré dans l'éther diéthylique. Les cristaux obtenus sont collectés par filtration et séchés sous vide. On obtient 0,12 g de produit.

Point de fusion : 269-271°C

RMN $^1H$ (DMSO) $\delta$ (ppm): 11.20 (1H, s); 9.10 (1H, s); 8.45 (1H, s); 8.35 (1H, d); 7.95 (1H, d); 3.70 (2H, s); 3.60-3.45 (2H, t); 3.30 (2H, d); 2.45-1.85 (6H, m).

Le composé n°18 a été préparé selon la méthode décrite dans l'exemple 4, à partir de (-)-5-(6-chloropyridazin-3-yl)-1-azabicyclo[3.2.1]octane, obtenu par dédoublement du mélange racémique (préparé à l'étape 1.5 de l'exemple 1) par chromatographie liquide sur support chiral.

**<u>Exemple 5</u> (composé N°24)**

Bromhydrate (2 : 1) de (-)-5-[6-(-1*H*-imidazol-2-yl)pyridazin-3-yl]-1-azabicyclo[3.2.1]-octane

Dans un tricol de 25ml on introduit 0,39g (2,23 mmoles) de 1-(diméthylaminosuffonyl)imidazole en solution dans 10 ml de tétrahydrofurane. Le milieu réactionnel est refroidi à -78°C et on ajoute 1,4 ml d'une solution 1,6M de n-butyllithium dans l'hexane, goutte à goutte, en 20 minutes. On ajoute ensuite 0,31g (2,32 mmoles) de chlorure de zinc en solution dans 4 ml de tétrahydrofurane. On agite en laissant la température remonter jusqu'à 20°C, puis on ajoute successivement 0,48g (3,58 mmoles) de chlorure de zinc, 0,06g (0,05 mmole) de tetrakis(triphénylphosphino)palladium et 0,2g (0,89 mmole) de (-)-5-(6-chloropyridazin-3-yl)-azabicyclo[3.2.1]octane (obtenu par dédoublement du mélange racémique préparé à l'étape 1.5 de l'exemple 1 par chromatographie liquide sur support chiral) en solution dans 5 ml de tétrahydrofurane. Le mélange est alors chauffé au reflux pendant 24 heures puis refroidi à température ambiante. On ajoute 30 ml d'une solution aqueuse d'hydroxyde de sodium à 30% et 50 ml de chloroforme. La phase aqueuse est extraite par du chloroforme puis les phases organiques réunies sont lavées par une solution aqueuse saturée de chlorure de sodium, séchées sur sulfate de sodium, filtrées et concentrées sous pression réduite.

Le résidu obtenu est mis en solution dans 10 ml de dioxane et 1,5 ml d'une solution aqueuse d'acide chlorhydrique 2N. Le milieu est agité à température ambiante pendant 2 heures puis le solvant est évaporé sous pression réduite. Le résidu est repris dans 30 ml de chloroforme et 30 ml d'une solution aqueuse saturée de carbonate de sodium. La phase aqueuse est extraite par du chloroforme et les phases organiques réunies sont séchées sur sulfate de sodium, filtrées et concentrées sous pression réduite. Le résidu est purifié par chromatographie sur colonne de gel de silice en éluant par un mélange de chloroforme, méthanol et ammoniaque dans les proportions 90/10/1. On obtient 0,033 g de produit attendu que l'on dissout dans 3 ml d'alcool isopropylique pour additionner 0,045 ml d'une solution d'acide bromhydrique 5,7N dans l'acide acétique. Les cristaux formés sont collectés par filtration et séchés sous vide. On obtient 0,027g de produit.

Point de fusion : 290-292°C

RMN $^1H$ (DMSO) $\delta$ (ppm): 10.15 (1H, s); 8.40 (1H, d); 8.05 (1H, d); 7.80 (2H, s); 3.80 (1H, s); 3.70-3.50 (2H, t); 3.35 (2H, d); 2.45-1.85 (6H, m).

Le tableau 1 qui suit illustre les structures chimiques et les propriétés physiques de quelques exemples de composés selon l'invention. Dans ce tableau :

- dans la colonne $[\alpha_D^{20}]$ ($CH_3OH$), la valeur indiquée représente le pouvoir rotatoire du composé, la concentration en g/100ml dans le méthanol à laquelle cette mesure a été réalisée étant indiquée entre parenthèses ; les composés sans mention dans cette colonne sont des racémates ;
- dans la colonne "Sel", "-" désigne un composé à l'état de base, "HBr" désigne un bromhydrate et "HCl" désigne un chlorhydrate. Les rapports molaires acide:base sont indiqués en regard.

**Tableau 1**

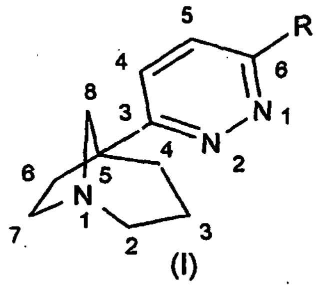

(I)

| N° | R | Sel | $[\alpha_D^{20}]$ ($CH_3OH$) | PF (°C) (Point de fusion) |
|---|---|---|---|---|
| **1** | $C_6H_5$ | - | - | 133-134 |
| **2** | 3-furyl | - | - | 140-141 |
| **3** | 4-$CH_3$-2-thiényl | - | - | 139-140 |
| **4** | Cl | HCl 1:1 | - | 234-235 |
| **5** | 5-$CH_3$-2-thiényl | HCl 1:1 | - | 245-246 |
| **6** | 3,5-$(CH_3)_2$-$C_6H_3$ | HCl 1:1 | - | 182-184 |
| **7** | 4-$OCH_3$-$C_6H_4$ | HCl 1:1 | - | 229-231 |
| **8** | 2-furyl | HCl 1:1 | - | 274-275 |
| **9** | 3,4-$(OCH_3)_2$-$C_6H_3$ | HCl 1:1 | - | 224-226 |
| **10** | 3-F-$C_6H_4$ | HCl 1:1 | - | 269-270 |
| **11** | 3-Cl-$C_6H_4$ | HCl 1:1 | - | 231-232 |
| **12** | 3,5-$(CH_3)_2$-4-pyrazolyl | HCl 1:1 | n.d.* Enantiomère(+) | 260-261 |
| **13** | 2-pyrrolyl | - | + 39,6 (c = 0,6) | 207-209 |
| **14** | 3-pyridinyl | - | - | 131-133 |
| **15** | 5-$CH_3$-2-thiényl | HCl 1:1 | -1.7 (c = 0,45) | 270-272 |
| **16** | 3-furyl | HCl 1:1 | + 14,1 (c = 0,3) | 226-228 |
| **17** | 5-$CH_3$-2-thiényl | HCl 1:1 | + 20,6 (c = 0,38) | 269-271 |
| **18** | 4-imidazolyl | HCl 2:1 | -18,2 (c = 1) | 271-273 |
| **19** | 4-imidazolyl | | HCl 2:1 15,8 (c = 1) | 269-271 |
| **20** | 4-pyrazolyl | HBr 2:1 | -17,1 (c = 1) | 221-223 |
| **21** | 1-$CH_3$-4-pyrazolyl | HBr 2:1 | - 1 (c = 1) | 273-275 |
| **22** | 4-pyrazolyl | HBr 2:1 | + 14,9 (c= 0,7) | 295-297 |
| **23** | 1-$CH_3$-4-pyrazolyl | HBr 2:1 | + 15,4 (c = 1) | 279-281 |
| **24** | 2-imidazolyl | HBr 2:1 | -15, 3 (c = 0,65) | 290-292 |
| **25** | 1-imidazolyl | - | + 63,6 (c = 0,2**) | 177-179 |

(suite)

| N° | R | Sel | $[\alpha_D^{20}]$ (CH$_3$OH) | PF (°C) (Point de fusion) |
|---|---|---|---|---|
| **26** | 1-imidazolyl | - | - 49,5 (c = 0,4) | 177-179 |
| * n.d. = valeur non déterminée<br>** concentration en g/100ml dans le DMSO | | | | |

Les composés de l'invention ont fait l'objet d'essais pharmacologiques qui ont mis en évidence leur intérêt comme substances actives de médicaments.

Ainsi ils ont été étudiés quant à leur affinité vis-à-vis des récepteurs nicotiniques contenant la sous unité $\alpha_7$, selon les méthodes décrites par Mark et Collins dans *J. Pharmacol. Exp. Ther.* 1982, **22,** 564 et par Marks et coll. dans *Mol. Pharmacol,* 1986, **30**, 427.

On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4 °C, puis on le centrifuge à 1000 G pendant 10 min. On élimine le culot et on centrifuge le surnageant à 8000 G pendant 20 min à 4 °C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [$^3$H]-$\alpha$-bungarotoxine à 1 nM dans un volume final de 250 µl de tampon HEPES 20 mM, polyéthylènimine 0,05%. On arrête la réaction par filtration sur des filtres Whatman GF/C™ préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de $\alpha$-bungarotoxine à 1 µM finale ; la liaison non spécifique représente, environ 60 % de la liaison totale récupérée sur le filtre. Pour chaque concentration de composé étudié on détermine le pourcentage d'inhibition de la liaison spécifique de [$^3$H]-$\alpha$-bungarotoxine, puis on calcule la $CI_{50}$, concentration de composé qui inhibe 50% de la liaison spécifique.

Les $CI_{50}$ des composés de l'invention les plus affins se situent entre 0,001 et 1 µM.

Les composés de l'invention ont aussi été étudiés quant à leur affinité vis à vis des récepteurs nicotiniques contenant la sous-unité $\alpha_4\beta_2$ selon les méthodes décrites par Anderson et Americ dans Eur. J. Pharmacol. 1994, 253, 261 et par Hall et coll, dans Brain Res. 1993, 600, 127.

On décapite des rats mâles Sprague Dawley de 150 à 200 g et on prélève rapidement la totalité du cerveau, on l'homogénéise dans 15 volumes d'une solution de sucrose à 0,32 M à 4°C, puis on le centrifuge à 1000 G pendant 10 min. On élimine le culot et centrifuge le surnageant à 20000 G pendant 20 min à 4°C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau (buffy coat) à 40000 G pendant 20 min, on récupère le culot, on le remet en suspension dans 15 ml d'eau bidistillée et on le centrifuge encore une fois à 40000 G avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min en présence de 100 µl de [$^3$H]-cytisine à 1 nM dans un volume final de 500 µl de tampon, en présence ou en absence de composé à tester. On arrête la réaction par filtration sur des filtres Whatman GF/B™ préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de [$^3$H]-cytisine, à des doses de 1 µM et 10 µM. Pour les composés les plus affins de l'invention, on calcule la $CI_{50}$, concentration de composé qui inhibe 50% de la liaison spécifique.

Les $CI_{50}$ des composés de l'invention les plus affins se situent entre 0,2 et 10 µM.

Les données expérimentales de quelques composés spécifiques sont indiquées dans le tableau 2 qui suit.

**Tableau 2**

| Composé N° | $CI_{50}$ $\alpha_7$ (μM) | Pourcentage d'inhibition de la liaison spécifique de [$^3$H]-cytisine à la dose de 1 μM, pour la sous unité $\alpha_4\beta_2$ (%) |
|---|---|---|
| 3 | 0,428 | 10 |
| 7 | 0,164 | 9 |
| 8 | 0,442 | 4 |

Les composés de l'invention ont également été étudiés quant à leur affinité vis-à-vis des récepteurs nicotiniques périphériques de type ganglionnaire selon la méthode décrite par Houghtling et coll, dans Mol. Pharmacol. 1995, 48, 280.

On décongèle des glandes surrénales de boeuf conservées à -80°C, et on les homogénéise à l'aide d'un broyeur Polytron™ dans 20 volumes de tampon Tris-HCl 50 mM à pH 7,4 et à 4°C, puis on les centrifuge à 35000 G pendant 10 min. On élimine le surnageant et on remet le culot en suspension dans 30 volumes de tampon Tris-HCl 50 mM à 4°C et on ré-homogénéise avant de re-centrifuger à 35000 G pendant 10 min. On reprend le dernier culot dans 10 volumes de tampon Tris-HCl à 4°C. On fait incuber 100 μl de membrane soit 10 mg de tissu frais à 24°C pendant 3h en présence de 50 μl de [$^3$H]-epibatidine 0,66 nM finale dans un volume final de 250 μl de tampon, en présence ou en absence de composé à tester. On arrête la réaction par dilution des échantillons avec du tampon Tris-HCl 50 μM pH 7,4 à 4°C puis on filtre sur des filtres Whatman GF/C™ préalablement traités pendant 3 heures avec de la polyéthylènimine à 0,5%. On - rince les filtres deux fois avec 5 ml de tampon et on mesure la radioactivité retenue sur le filtre par scintigraphie liquide. On détermine la liaison non spécifique en présence de (-)-nicotine 2 mM finale; la liaison non spécifique représente 30 à 40% de la liaison totale récupérée sur le filtre. Pour chaque concentration de produit étudié on détermine le pourcentage d'inhibition de la liaison spécifique de [$^3$H]-epibatidine, puis on calcule la $CI_{50}$, concentration de composé qui inhibe 50% de la liaison spécifique.

Les $CI_{50}$ des composés de l'invention se situent entre 1 et 10 μM.

Les résultats obtenus montrent que certains composés de l'invention sont des ligands sélectif pour la sous-unité $\alpha_7$ du récepteur nicotinique et que d'autres sont mixtes $\alpha_4\beta_2$ et $\alpha_7$.

Ces résultats suggèrent l'utilisation des composés dans le traitement ou la prévention des désordres liés à un dysfonctionnement des récepteurs nicotiniques, notamment au niveau du système nerveux central.

Ces désordres comprennent les altérations cognitives, plus, spécifiquement mnésiques (acquisition, consolidation et rappel), mais également les atteintes des processus attentionnels, et les troubles des fonctions exécutives liées à la maladie d'Alzheimer, au vieillissement pathologique (Age Associated Memory Impairment,AAMI) ou normal (senile dementia), au syndrome Parkinsonien, à la trisomie 21 (Down's syndrome), aux pathologies psychiatriques (en particulier les troubles cognitifs associés à la schizophrénie), au syndrome alcoolique de Korsakoff, aux démences vasculaires (multi-infarct dementia, MDI), aux traumatismes crâniens.

Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.

Ils peuvent aussi présenter une activité thérapeutique neuro-protectrice vis à vis des atteintes anatomo-histopathologiques liées aux maladies neuro-dégénératives susnommées.

Les composés de l'invention peuvent également constituer un traitement curatif ou symptomatique des accidents vasculaires cérébraux et des épisodes hypoxiques cérébraux. Ils peuvent être utilisés dans les cas de pathologies psychiatriques : schizophrénie (symptômes positifs et/ou négatifs), troubles bipolaires, dépression, anxiété, attaques de panique, troubles de l'attention avec hyperactivité, comportements compulsifs et obsessionnels.

Ils peuvent prévenir les symptômes dus au sevrage au tabac, à l'alcool, aux différentes substances induisant une dépendance, telles que cocaïne, LSD, cannabis, benzodiazépines.

Ils peuvent être utiles dans le traitement de la douleur d'origine diverse (y compris les douleurs chroniques, neuropathiques ou inflammatoires).

Par ailleurs les composés de l'invention peuvent être utilisés pour le traitement de l'ischémie des membres inférieurs, de l'artérite oblitérante des membres inférieurs (PAD : peripheral arterial disease), de l'ischémie cardiaque (angor stable), de l'infarctus du myocarde, de l'insuffisance cardiaque, du déficit de cicatrisation cutanée des patients diabétiques, des ulcères variqueux de l'insuffisance veineuse.

Les composés de l'invention peuvent aussi être utilisés pour le traitement des processus inflammatoires d'origines diverses, en particulier les inflammations concernant le système nerveux central.

Les composés selon invention peuvent donc être utilisés pour la préparation de médicaments, en particulier de médicaments utiles dans le traitement ou la prévention des désordres liés à un dysfonctionnement des récepteurs

nicotiniques, notamment des désordres ci-dessus mentionnés.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, notamment dans le traitement ou la prévention des désordres liés à un dysfonctionnement des récepteurs nicotiniques, notamment des désordres ci-dessus mentionnés.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I)

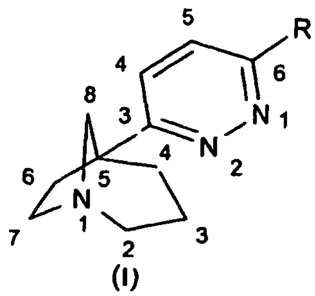

(I)

dans laquelle :

R représente
soit un atome d'hydrogène ou d'halogène ;
soit un groupe phényle éventuellement substitué par un ou plusieurs atomes d'halogène, par un ou plusieurs groupes choisis parmi les groupes ($C_1$-$C_6$)alkyle, ($C_1$-$C_6$)alcoxy, nitro, amino, di($C_1$-$C_3$)alkylamino, trifluorométhyle, trifluorométhoxy, cyano, hydroxy, acétyle ou méthylènedioxy ;
soit un groupe choisi parmi un pyridinyle, pyrazolyle, imidazolyle, triazolyle, tétrazolyle, oxazolyle, thiazolyle, oxadiazolyle, thiadiazolyle, thiényle, furyle, isoxazolyle, isothiazolyle, pyrrolyle, naphtyle, ce groupe pouvant éventuellement être substitué par un ou plusieurs groupes choisis parmi les atomes d'halogènes, les groupes ($C_1$-$C_6$)alkyle, ($C_1$-$C_6$)alcoxy, trifluorométhoxy, trifluorométhyle, nitro, cyano, hydroxy, amino, ($C_1$-$C_6$)alkylamino ou di($C_1$-$C_6$)alkylamino ;
à l'état de base ou de sel d'addition à un acide, ainsi qu'à l'état d'hydrate ou de solvat.

2. Composé de formule (I) selon la revendication 1, **caractérisé en ce que** R représente
soit un atome d'halogène ;
soit un groupe phényle éventuellement substitué par un ou plusieurs atomes d'halogène, par un ou plusieurs groupes choisis parmi les groupes ($C_1$-$C_6$)alkyle, ($C_1$-$C_6$)alcoxy ;
soit un groupe choisi parmi un pyridinyle, pyrazolyle, imidazolyle, triazolyle, tétrazolyle, oxazolyle, thiazolyle, oxadiazolyle, thiadiazolyle, thiényle, furyle, isoxazolyle, isothiazolyle, pyrrolyle, naphtyle, ce groupe pouvant éventuellement être substitué par un ou plusieurs groupes ($C_1$-$C_6$)alkyle ;
à l'état de base ou de sel d'addition à un acide, ainsi qu'à l'état d'hydrate ou de solvat.

3. Composé de formule (I) selon la revendication 1 ou 2, **caractérisé en ce que** R représente
soit un atome d'halogène ;
soit un groupe phényle éventuellement substitué par un ou plusieurs atomes d'halogène, par un ou plusieurs groupes choisis parmi les groupes ($C_1$-$C_6$)alkyle, ($C_1$-$C_6$)alcoxy;
soit un groupe choisi parmi un pyridinyle, pyrazolyle, imidazolyle, thiényle, furyle, pyrrolyle, ce groupe pouvant éventuellement être substitué par un ou plusieurs groupes ($C_1$-$C_6$)alkyle ; à l'état de base ou de sel d'addition à un acide, ainsi qu'à l'état d'hydrate ou de solvat.

4. Composé de formule (I) selon l'une des revendications 1 à 3, **caractérisé en ce qu'**il est choisi parmi :

- 5-(6-phényl-pyridazin-3-yl)-1-azabicyclo[3.2.1]octane
- 5-(6-(3-furyl)pyridazin-3-yl)-1-azabicyclo[3.2.1]octane
- 5-(6-(4-methyl-2-thienyl)pyridazin-3-yl)-1-azabicyclo[3.2.1]octane
- Chlorhydrate (1:1) de 5-(6-chloro-pyridazin-3-yl)-1-azabicyclo[3.2.1]octane
- Chlorhydrate (1:1) de 5-(6-(5-méthyl-2-thiényl)pyridazin-3-yl)-1-azabicyclo[3.2.1]octane
- Chlorhydrate (1 :1) de 5-(6-(3,5-diméthyl)phényl-pyridazin-3-yl)-1-azabicyclo[3.2.1]octane
- Chlorhydrate (1:1) de 5-(6-(4-méthoxy)phényl-pyridazin-3-yl)-1-azabicyclo[3.2.1]octane
- Chlorhydrate (1 :1) de 5-(6-(2-furyl)pyridazin-3-yl)-1-azabicyclo[3.2.1]octane
- Chlorhydrate (1 :1) de 5-(6-(3,4-diméthoxy)phényl-pyridazin-3-yl)-1-azabicyclo[3.2.1]octane
- Chlorhydrate (1 :1) de 5-(6-(3-fluoro)phényi-pyridazin-3-yl)-1-azabicyclo[3.2.1]octane
- Chlorhydrate (1 :1) de 5-(6-(3-chloro)phényl-pyridazin-3-yl)-1-azabicyclo[3.2.1]octane
- (+) Chlorhydrate (1:1) de 5-(6-(3,5-diméthyl-4-pyrazolyl)pyridazin-3-yl)-1-azabicyclo[3.2.1]octane
- (+) 5-(6-(1H-pyrrol-2-yl)pyridazin-3-yl)-1-azabicyclo[3.2.1]octane

- 5-(6-(1H-pyridin-3-yl)pyridazin-3-yl)-1-azabicyclo[3.2.1]octane
- (-) Chlorhydrate (1 :1) de 5-(6-(5-méthyl-2-thiényl)pyridazin-3-yl)-1-azabicyclo[3.2.1]octane
- (+) Chlorhydrate (1 :1) de 5-(6-(3-furyl)pyridazin-3-yl)-1-azabicyclo[3.2.1]octane
- (+) Chlorhydrate (1 :1) de 5-(6-(5-méthyl-2-thiényl)pyridazin-3-yl)-1-azabicyclo[3.2.1]octane
- (-) Chlorhydrate (1 :1) de 5-(6-(1H-imidazol-4-yl)pyridazin-3-yl)-1-azabicyclo[3.2.1]octane
- (+) Chlorhydrate (1 :1) de 5-(6-(1H-imidazol-4-yl)pyridazin-3-yl)-1-azabicyclo[3.2.1]octane
- (-) Bromhydrate (2:1) de 5-(6-(1H-pyrazol-4-yl)pyridazin-3-yl)-1-azabicyclo[3.2.1]octane
- (-) Bromhydrate (2:1) de 5-(6-(1-méthyl-1H-pyrazol-4-yl)pyridazin-3-yl)-1-azabicyclo[3.2.1]octane
- (+) Bromhydrate (2:1) de 5-(6-(1H-pyrazol-4yl)pyridazin-3-yl)-1-azabicyclo[3.2.1]octane
- (+) Bromhydrate (2:1) de 5-(6-(1-méthyl-1H-pyrazol-4-yl)pyridazin-3-yl)-1-azabicyclo[3.2.1]octane
- (-) Bromhydrate (2:1) de 5-(6-(-1H-imidazol-2-yl)pyridazin-3-yl)-1-azabicyclo[3.2.1]octane
- (+) 5-(6-(1H-imidazol-1-yl)pyridazin-3-yl)-1-azabicyclo[3.2.1]octane
- (-) 5-(6-(1H-imidazol-1-yl)pyridazin-3-yl)-1-azabicyclo[3.2.1]octane

**5.** Procédé de préparation d'un composé de formule (I) selon l'une quelconque des revendications 1 à 4, **caractérisé en ce que** l'on fait réagir le composé de formule (VII)

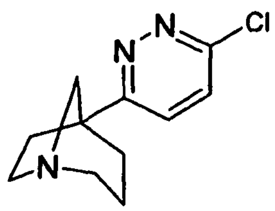

(VII)

soit avec un acide boronique de formule $R-B(OH)_2$ dans laquelle R est tel que défini dans la formule générale (I), en présence d'un catalyseur au palladium ;
soit avec un composé de formule R-H dans laquelle R est tel que défini dans la formule générale (I), en présence d'une base forte dans un solvant ;
soit avec un dérivé stanneux de formule $R-Sn[(CH_2)_3CH_3)]_3$ dans laquelle R est tel que défini dans la formule générale (I), en présence d'un catalyseur au palladium ;
soit avec un composé de formule R-H dans laquelle R est tel que défini dans la formule générale (I), en présence de n-butyllithium, de chlorure de zinc et d'un catalyseur au palladium.

**6.** Médicament, **caractérisé en ce qu'**il comprend un composé de formule (I) selon l'une quelconque des revendications 1 à 4, ou un sel d'addition de ce composé à un acide pharmaceutiquement acceptable, ou encore un hydrate ou un solvat.

**7.** Composition pharmaceutique, **caractérisée en ce qu'**elle comprend un composé de formule (I) selon l'une quelconque des revendications 1 à 4, ou un sel pharmaceutiquement acceptable, un hydrate ou un solvat de ce composé, ainsi qu'au moins un excipient pharmaceutiquement acceptable.

**8.** Utilisation d'un composé de formule (I) selon l'une quelconque des revendications 1 à 4 pour la préparation d'un médicament destiné au traitement et à la prévention des altérations cognitives ; des atteintes des processus attentionnels ; des troubles des fonctions exécutives liées à la maladie d'Alzheimer, au vieillissement pathologique ou normal, au syndrome Parkinsonien, à la trisomie 21, aux pathologies psychiatriques, au syndrome alcoolique de Korsakoff, aux démences vasculaires, aux traumatismes crâniens ; des troubles moteurs observés dans la maladie de Parkinson ou d'autres maladies neurologiques ou des atteintes anatomo-histo-pathologiques liées aux maladies neuro-dégénératives susnommées.

**9.** Utilisation d'un composé de formule (I) selon l'une quelconque des revendications 1 à 4 pour la préparation d'un médicament destiné au traitement et à la prévention des accidents vasculaires cérébraux, des épisodes hypoxiques cérébraux, des pathologies psychiatriques.

**10.** Utilisation d'un composé de formule (I) selon l'une quelconque des revendications 1 à 4 pour la préparation d'un médicament destiné à la prévention des symptômes dus au sevrage au tabac, à l'alcool, aux différentes substances induisant une dépendance.

11. Utilisation d'un composé de formule (I) selon l'une quelconque des revendications 1 à 1 pour la préparation d'un médicament destiné au traitement de la douleur.

12. Utilisation d'un composé de formule (I) selon l'une quelconque des revendications 1 à 4 pour la préparation d'un médicament destiné au traitement de l'ischémie des membres inférieurs, de l'artérite oblitérante des membres inférieurs, de l'ischémie cardiaque, de l'infarctus du myocarde, de l'insuffisance cardiaque, du déficit de cicatrisation cutanée des patients diabétiques, des ulcères variqueux de l'insuffisance veineuse.

13. Utilisation d'un composé de formule (I) selon l'une quelconque des revendications 1 à 4 pour la préparation d'un médicament destiné au traitement des processus inflammatoires.

14. Composé de formule (I) selon l'une des revendications 1 à 4 pour la préparation d'un médicament destiné au traitement et à la prévention des altérations cognitives ; des atteintes des processus attentionnels ; des troubles des fonctions exécutives liées à la maladie d'Alzheimer, au vieillissement pathologique ou normal, au syndrome Parkinsonien, à la trisomie 21, aux pathologies psychiatriques, au syndrome alcoolique de Korsakoff, aux démences vasculaires, aux traumatismes crâniens ; des troubles moteurs observés dans la maladie de Parkinson ou d'autres maladies neurologiques ou des atteintes anatomo-histo-pathologiques liées aux maladies neuro-dégénératives susnommées.

15. Composé de formule (I) selon l'une des revendications 1 à 4 pour la préparation d'un médicament destiné au traitement et à la prévention des accidents vasculaires cérébraux, des épisodes hypoxiques cérébraux, des pathologies psychiatriques.

16. Composé de formule (I) selon l'une des revendications 1 à 4 pour la préparation d'un médicament destiné à la prévention des symptômes dus au sevrage au tabac, à l'alcool, aux différentes substances induisant une dépendance.

17. Composé de formule (I) selon l'une des revendications 1 à 4 pour la préparation d'un médicament destiné au traitement de la douleur.

18. Composé de formule (I) selon l'une des revendications 1 à 4 pour la préparation d'un médicament destiné au traitement de l'ischémie des membres inférieurs, de l'artérite oblitérante des membres inférieurs, de l'ischémie cardiaque, de l'infarctus du myocarde, de l'insuffisance cardiaque, du déficit de cicatrisation cutanée des patients diabétiques, des ulcères variqueux de l'insuffisance veineuse.

19. Composé de formule (I) selon l'une des revendications 1 à 4 pour la préparation d'un médicament destiné au traitement des processus inflammatoires.

## Claims

1. Compound corresponding to formula (I)

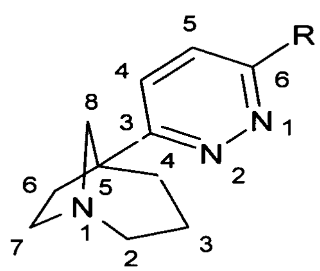

(I)

in which:

R is
either a hydrogen or halogen atom;
or a phenyl group optionally substituted with one or more halogen atoms, or with one or more groups selected from ($C_1$-$C_6$)alkyl, ($C_1$-$C_6$) alkoxy, nitro, amino, di($C_1$-$C_3$)alkylamino, trifluoromethyl, trifluoromethoxy, cyano, hydroxyl, acetyl or methylenedioxy groups;
or a group selected from a pyridinyl, pyrazolyl, imidazolyl, triazolyl, tetrazolyl, oxazolyl, thiazolyl, oxadiazolyl, thiadiazolyl, thienyl, furyl, isoxazolyl, isothiazolyl, pyrrolyl and naphthyl, it being possible for this group to be optionally substituted with one or more groups selected from halogen atoms, and ($C_1$-$C_6$) alkyl, ($C_1$-$C_6$) alkoxy, trifluoromethoxy, trifluoromethyl, nitro, cyano, hydroxyl, amino, ($C_1$-$C_6$) alkylamino or di($C_1$-$C_6$)alkylamino groups;
in the form of a base or of an addition salt with an acid, and also in the form of a hydrate or of a solvate.

2. Compound of formula (I) according to Claim 1, **characterized in that**
R is
either a halogen atom,
or a phenyl group optionally substituted with one or more halogen atoms, or with one or more groups selected from ($C_1$-$C_6$) alkyl and ($C_1$-$C_6$) alkoxy groups;
or a group selected from a pyridinyl, pyrazolyl, imidazolyl, triazolyl, tetrazolyl, oxazolyl, thiazolyl, oxadiazolyl, thiadiazolyl, thienyl, furyl, isoxazolyl, isothiazolyl, pyrrolyl and naphthyl, it being possible for this group to be optionally substituted with one or more ($C_1$-$C_6$) alkyl groups,
in the form of a base or of an addition salt with an acid, and also in the form of a hydrate or of a solvate.

3. Compound of formula (I) according to Claim 1 or 2, **characterized in that**
R is
either a halogen atom;
or a phenyl group optionally substituted with one or more halogen atoms, or with one or more groups selected from ($C_1$-$C_6$) alkyl and ($C_1$-$C_6$) alkoxy groups;
or a group selected from a pyridinyl, pyrazolyl, imidazolyl, thienyl, furyl and pyrrolyl, it being possible for this group to be optionally substituted with one or more ($C_1$-$C_6$)alkyl groups;
in the form of a base or of an addition salt with an acid, and also in the form of a hydrate or of a solvate.

4. Compound of formula (I) according to one of Claims 1 to 3, **characterized in that** it is selected from:

- 5-(6-phenylpyridazin-3-yl)-1-azabicyclo[3.2.1]octane
- 5-(6-(3-furyl)pyridazin-3-yl)-1-azabicyclo[3.2.1]-octane
- 5-(6-(4-methyl-2-thienyl)pyridazin-3-yl)-1-azabicyclo[3.2.1]octane
- 5-(6-chloropyridazin-3-yl)-1-azabicyclo[3.2.1]octane hydrochloride (1:1)
- 5-(6-(5-methyl-2-thienyl)pyridazin-3-yl)-1-azabicyclo[3.2.1]octane hydrochloride (1:1)
- 5-(6-(3,5-dimethyl)phenylpyridazin-3-yl)-1-azabicyclo[3.2.1]octane hydrochloride (1:1)
- 5-(6-(4-methoxy)phenylpyridazin-3-yl)-1-azabicyclo-[3.2.1]octane hydrochloride (1:1)
- 5-(6-(2-furyl)pyridazin-3-yl)-1-azabicyclo[3.2.1]-octane hydrochloride (1:1)
- 5-(6-(3,4-dimethoxy)phenylpyridazin-3-yl)-1-azabicyclo[3.2.1]octane hydrochloride (1:1)
- 5-(6-(3-fluoro)phenylpyridazin-3-yl)-1-azabicyclo-[3.2.1]octane hydrochloride (1:1)
- 5-(6-(3-chloro)phenylpyridazin-3-yl)-1-azabicyclo-[3.2.1]octane hydrochloride (1:1)
- (+)-5-(6-(3,5-dimethyl-4-pyrazolyl)pyridazin-3-yl)-1-azabicyclo[3.2.1]octane hydrochloride (1:1)
- (+)-5-(6-(1H-pyrrol-2-yl)pyridazin-3-yl)-1-azabicyclo[3.2.1]octane
- 5-(6-(1H-pyridin-3-yl)pyridazin-3-yl)-1-azabicyclo-[3.2.1]octane
- (-)-5-(6-(5-methyl-2-thienyl)pyridazin-3-yl)-1-azabicyclo[3.2.1]octane hydrochloride (1:1)
- (+)-5-(6-(3-furyl)pyridazin-3-yl)-1-azabicyclo-[3.2.1]octane hydrochloride (1:1)
- (+)-5-(6-(5-methyl-2-thienyl)pyridazin-3-yl)-1-azabicyclo[3.2.1]octane hydrochloride (1:1)
- (-)-5-(6-(1H-imidazol-4-yl)pyridazin-3-yl)-1-azabicyclo[3.2.1]octane hydrochloride (1:1)
- (+)-5-(6-(1H-imidazol-4-yl)pyridazin-3-yl)-1-azabicyclo[3.2.1]octane hydrochloride (1:1)
- (-)-5-(6-(1H-pyrazol-4-yl)pyridazin-3-yl)-1-azabicyclo[3.2.1]octane hydrobromide (1:2)
- (-)-5-(6-(1-methyl-1H-pyrazol-4-yl)pyridazin-3-yl)-1-azabicyclo[3.2.1]octane hydrobromide (1:2)
- (+)-5-(6-(1H-pyrazol-4-yl)pyridazin-3-yl)-1-azabicyclo[3.2.1]octane hydrobromide (1:2)
- (+)-5-(6-(1-methyl-1H-pyrazol-4-yl)pyridazin-3-yl)-1-azabicyclo[3.2.1]octane hydrobromide (1:2)
- (-)-5-(6-(1H-imidazol-2-yl)pyridazin-3-yl)-1-azabicyclo[3.2.1]octane hydrobromide (1:2)
- (+)-5-(6-(1H-imidazol-1-yl)pyridazin-3-yl)-1-azabicyclo[3.2.1]octane

- (-)-5-(6-(1H-imidazol-1-yl)pyridazin-3-yl)-1-azabicyclo[3.2.1]octane

5. Process for preparing a compound of formula (I) according to any one of Claims 1 to 4, **characterized in that** the compound of formula (VII)

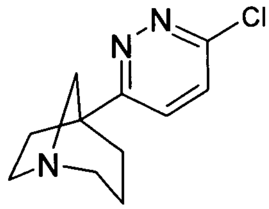

(VII)

is reacted
either with a boronic acid of formula $R-B(OH)_2$ in which R is as defined in formula (I), in the presence of a palladium catalyst;
or with a compound of formula R-H in which R is as defined in formula (I), in the presence of a strong base in a solvent;
or with a stannous derivative of formula $R-Sn[(CH_2)_3CH_3)]_3$ in which R is as defined in formula (I), in the presence of a palladium catalyst;
or with a compound of formula R-H in which R is as defined in formula (I), in the presence of n-butyllithium, of zinc chloride and of a palladium catalyst.

6. Medicament, **characterized in that** it comprises a compound of formula (I) according to any one of Claims 1 to 4, or an addition salt of this compound with a pharmaceutically acceptable acid, or else a hydrate or a solvate.

7. Pharmaceutical composition, **characterized in that** it comprises a compound of formula (I) according to any one of Claims 1 to 4, or a pharmaceutically acceptable salt, a hydrate or a solvate of this compound, and also at least one pharmaceutically acceptable excipient.

8. Use of a compound of formula (I) according to any one of Claims 1 to 4, for the preparation of a medicament for use in the treatment and prevention of cognitive impairments; of affected attention processes; of problems with executive functions related to Alzheimer's disease, to pathological ageing or normal ageing, to Parkinsonian syndrome, to trisomy 21, to psychiatric pathologies, to alcoholic Korsakoff's syndrome, to vascular dementias, to cranial traumas; of motor problems observed in Parkinson's disease or other neurological diseases or of anatomical-histopathological damage related to the abovementioned neurodegenerative diseases.

9. Use of a compound of formula (I) according to any one of Claims 1 to 4, for the preparation of a medicament for use in the treatment and prevention of cerebral strokes, of hypoxic episodes in the brain, or of psychiatric pathologies.

10. Use of a compound of formula (I) according to any one of Claims 1 to 4, for the preparation of a medicament for use in the prevention of symptoms due to tobacco withdrawal, alcohol withdrawal, or withdrawal from the various substances which induce dependency.

11. Use of a compound of formula (I) according to any one of Claims 1 to 4, for the preparation of a medicament for use in the treatment of pain.

12. Use of a compound of formula (I) according to any one of Claims 1 to 4, for the preparation of a medicament for use in the treatment of lower limb ischaemia, of obliterative arteritis of the lower limbs, of cardiac ischaemia, of myocardial infarction, of heart failure, of cutaneous cicatrisation deficiency in diabetic patients, and of varicose ulcers in venous insufficiency.

13. Use of a compound of formula (I) according to any one of Claims 1 to 4, for the preparation of a medicament for use in the treatment of inflammatory processes.

14. Compound of formula (I) according to one of Claims 1 to 4, for the preparation of a medicament for use in the treatment and prevention of cognitive impairments; of affected attention processes; of problems with executive functions related to Alzheimer's disease, to pathological ageing or normal ageing, to Parkinsonian syndrome, to

trisomy 21, to psychiatric pathologies, to alcoholic Korsakoff's syndrome, to vascular dementias, to cranial traumas; of motor problems observed in Parkinson's disease or other neurological diseases or of anatomical-histopathological damage related to the abovementioned neurodegenerative diseases.

**15.** Compound of formula (I) according to one of Claims 1 to 4, for the preparation of a medicament for use in the treatment and prevention of cerebral strokes, of hypoxic episodes in the brain, or of psychiatric pathologies.

**16.** Compound of formula (I) according to one of Claims 1 to 4, for the preparation of a medicament for use in the prevention of symptoms due to tobacco withdrawal, alcohol withdrawal, or withdrawal from the various substances which induce dependency.

**17.** Compound of formula (I) according to one of Claims 1 to 4, for the preparation of a medicament for use in the treatment of pain.

**18.** Compound of formula (I) according to one of Claims 1 to 4, for the preparation of a medicament for use in the treatment of lower limb ischaemia, of obliterative arteritis of the lower limbs, of cardiac ischaemia, of myocardial infarction, of heart failure, of cutaneous cicatrisation deficiency in diabetic patients, and of varicose ulcers in venous insufficiency.

**19.** Compound of formula (I) according to one of Claims 1 to 4, for the preparation of a medicament for use in the treatment of inflammatory processes.

## Patentansprüche

**1.** Verbindung der Formel (I)

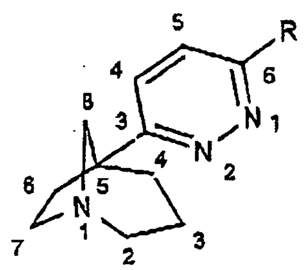

(I)

worin:

R für
ein Wasserstoff- oder Halogenatom;
eine Phenylgruppe, die gegebenenfalls durch ein oder mehrere Halogenatome und/oder durch eine oder mehrere unter ($C_1$-$C_6$) -Alkyl-, ($C_1$-$C_6$) -Alkoxy-, Nitro-, Amino-, Di-($C_1$-$C_3$)-alkylamino-, Trifluormethyl-, Trifluormethoxy-, Cyano-, Hydroxy-, Acetyl- und Methylendioxygruppen ausgewählte Gruppen substituiert ist;
oder eine unter Pyridinyl, Pyrazolyl, Imidazolyl, Triazolyl, Tetrazolyl, Oxazolyl, Thiazolyl, Oxadiazolyl, Thiadiazolyl, Thienyl, Furyl, Isoxazolyl, Isothiazolyl, Pyrrolyl und Naphthyl ausgewählte Gruppe, die gegebenenfalls durch eine oder mehrere unter Halogenatomen und ($C_1$-$C_6$)-Alkyl-, ($C_1$-$C_6$)-Alkoxy-, Trifluormethoxy-, Trifluormethyl-, Nitro-, Cyano-, Hydroxy-, Amino-, ($C_1$-$C_6$) -Alkylamino- und Di-($C_1$-$C_6$)-alkylaminogruppen ausgewählte Gruppen substituiert sein kann; steht;
in Basen- oder Säureadditionssalzform sowie in Hydrat- oder Solvatform.

**2.** Verbindung der Formel (I) nach Anspruch 1, **dadurch gekennzeichnet, daß**
R für
ein Halogenatom;
eine Phenylgruppe, die gegebenenfalls durch ein oder mehrere Halogenatome und/oder durch eine oder mehrere

unter ($C_1$-$C_6$) -Alkyl- und ($C_1$-$C_6$) -Alkoxygruppen ausgewählte Gruppen substituiert ist; oder eine unter Pyridinyl, Pyrazolyl, Imidazolyl, Triazolyl, Tetrazolyl, Oxazolyl, Thiazolyl, Oxadiazolyl, Thiadiazolyl, Thienyl, Furyl, Isoxazolyl, Isothiazolyl, Pyrrolyl und Naphthyl ausgewählte Gruppe, die gegebenenfalls durch eine oder mehrere ($C_1$-$C_6$)-Alkylgruppen substituiert sein kann;
steht;
in Basen- oder Säureadditionssalzform sowie in Hydrat- oder Solvatform.

**3.** Verbindung der Formel (I) nach Anspruch 1 oder 2, **dadurch gekennzeichnet, daß**
R für
ein Halogenatom;
eine Phenylgruppe, die gegebenenfalls durch ein oder mehrere Halogenatome und/oder durch eine oder mehrere unter ($C_1$-$C_6$)-Alkyl- und ($C_1$-$C_6$)-Alkoxygruppen ausgewählte Gruppen substituiert ist;
oder eine unter Pyridinyl, Pyrazolyl, Imidazolyl, Thienyl, Furyl und Pyrrolyl ausgewählte Gruppe, die gegebenenfalls durch eine oder mehrere ($C_1$-$C_6$)-Alkylgruppen substituiert sein kann;
steht;
in Basen- oder Säureadditionssalzform sowie in Hydrat- oder Solvatform.

**4.** Verbindung der Formel (I) nach einem der Ansprüche 1 bis 3, **dadurch gekennzeichnet, daß** sie unter:

- 5-(6-Phenylpyridazin-3-yl)-1-azabicyclo[3.2.1]-octan
- 5-(6-(3-Furyl)pyridazin-3-yl)-1-azabicyclo-[3.2.1]octan
- 5-(6-(4-Methyl-2-thienyl)pyridazin-3-yl)-1-azabicyclo[3.2.1]octan
- 5-(6-Chlorpyridazin-3-yl)-1-azabicyclo[3.2.1]-octan-hydrochlorid (1:1)
- 5-(6-(5-Methyl-2-thienyl)pyridazin-3-yl)-1-azabicyclo[3.2.1]octan-hydrochlorid (1:1)
- 5-(6-(3,5-Dimethyl)phenylpyridazin-3-yl)-1-azabicyclo[3.2.1]octan-hydrochlorid (1:1)
- 5-(6-(4-Methoxy)phenylpyridazin-3-yl)-1-azabicyclo[3.2.1]octan-hydrochlorid (1:1)
- 5-(6-(2-Furyl)pyridazin-3-yl)-1-azabicyclo-[3.2.1]octan-hydrochlorid (1:1)
- 5-(6-(3,4-Dimethoxy)phenylpyridazin-3-yl)-1-azabicyclo[3.2.1]octan-hydrochlorid (1:1)
- 5-(6-(3-Fluor)phenylpyridazin-3-yl)-1-azabicyclo[3.2.1]octan-hydrochlorid (1:1)
- 5-(6-(3-Chlor)phenylpyridazin-3-yl)-1-azabicyclo[3.2.1]octan-hydrochlorid (1:1)
- (+)-5-(6-(3,5-Dimethyl-4-pyrazolyl)pyridazin-3-yl)-1-azabicyclo[3.2.1]octan-hydrochlorid (1:1)
- (+)-5-(6-(1H-Pyrrol-2-yl)pyridazin-3-yl)-1-azabicyclo[3.2.1]octan
- 5-(6-(1H-Pyridin-3-yl)pyridazin-3-yl)-1-azabicyclo[3.2.1]octan
- (-)-5-(6-(5-Methyl-2-thienyl)pyridazin-3-yl)-1-azabicyclo[3.2.1]octan-hydrochlorid (1:1)
- (+)-5-(6-(3-Furyl)pyridazin-3-yl)-1-azabicyclo-[3.2.1]octan-hydrochlorid (1:1)
- (+)-5-(6-(5-Methyl-2-thienyl)pyridazin-3-yl)-1-azabicyclo[3.2.1]octan-hydrochlorid (1:1)
- (-)-5-(6-(1H-Imidazol-4-yl)pyridazin-3-yl)-1-azabicyclo[3.2.1]octan-hydrochlorid (1:1)
- (+)-5-(6-(1H-Imidazol-4-yl)pyridazin-3-yl)-1-azabicyclo[3.2.1]octan-hydrochlorid (1:1)
- (-)-5-(6-(1H-Pyrazol-4-yl)pyridazin-3-yl)-1-azabicyclo[3.2.1]octan-hydrobromid (1:2)
- (-)-5-(6-(1-Methyl-1H-pyrazol-4-yl)pyridazin-3-yl)-1-azabicyclo[3.2.1]octan-hydrobromid (1:2)
- (+)-5-(6-(1H-Pyrazol-4-yl)pyridazin-3-yl)-1-azabicyclo[3.2.1]octan-hydrobromid (1:2)
- (+)-5-(6-(1-Methyl-1H-pyrazol-4-yl)pyridazin-3-yl)-1-azabicyclo[3.2.1]octan-hydrobromid (1:2)
- (-)-5-(6-(1H-Imidazol-2-yl)pyridazin-3-yl)-1-azabicyclo[3.2.1]octan-hydrobromid (1:2)
- (+)-5-(6-(1H-Imidazol-1-yl)pyridazin-3-yl)-1-azabicyclo[3.2.1]octan
- (-)-5-(6-(1H-Imidazol-1-yl)pyridazin-3-yl)-1-azabicyclo[3.2.1]octan

ausgewählt ist.

**5.** Verfahren zur Herstellung einer Verbindung der Formel (I) nach einem der Ansprüche 1 bis 4, **dadurch gekennzeichnet, daß** man die Verbindung der Formel (VII)

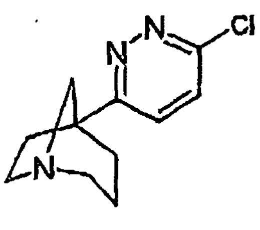

(VII)

in Gegenwart eines Palladiumkatalysators mit einer Boronsäure der Formel R-B$(OH)_2$, worin R die in der allgemeinen Formel (I) angegebene Bedeutung besitzt;
in Gegenwart einer starken Base in einem Lösungsmittel mit einer Verbindung der Formel R-H, worin R die in der allgemeinen Formel (I) angegebene Bedeutung besitzt;
in Gegenwart eines Palladiumkatalysators mit einem Zinnderivat der Formel R-Sn$[(CH_2)_3CH_3)]_3$, worin R die in der allgemeinen Formel (I) angegebene Bedeutung besitzt;
oder in Gegenwart von n-Butyllithium, Zinkchlorid und einem Palladiumkatalysator mit einer Verbindung der Formel R-H, worin R die in der allgemeinen Formel (I) angegebene Bedeutung besitzt;
umsetzt.

**6.** Arzneimittel, **dadurch gekennzeichnet, daß** es eine Verbindung der Formel (I) nach einem der Ansprüche 1 bis 4 oder ein Additionssalz dieser Verbindung mit einer pharmazeutisch unbedenklichen Säure oder auch ein Hydrat oder ein Solvat enthält.

**7.** Pharmazeutische Zusammensetzung, **dadurch gekennzeichnet, daß** sie eine Verbindung der Formel (I) nach einem der Ansprüche 1 bis 4 oder ein pharmazeutisch unbedenkliches Salz, ein Hydrat oder ein Solvat dieser Verbindung sowie mindestens einen pharmazeutisch unbedenklichen Hilfsstoff enthält.

**8.** Verwendung einer Verbindung der Formel (I) nach einem der Ansprüche 1 bis 4 zur Herstellung eines Arzneimittels zur Behandlung und Prävention von kognitiven Störungen; Aufmerksamkeitsstörungen; Störungen der exekutiven Funktionen in Verbindung mit Alzheimer-Krankheit, pathologischer oder normaler Alterung, Parkinson-Syndrom, Trisomie 21, psychiatrischen Pathologien, Korsakoff-Alkoholsyndrom, vaskulären Demenzen oder Schädeltraumen; motorischen Störungen, die bei Parkinson-Krankheit oder anderen neurologischen Erkrankungen beobachtet werden, oder anatomisch-histopathologischen Störungen in Verbindung mit den oben aufgeführten neurodegenerativen Erkrankungen.

**9.** Verwendung einer Verbindung der Formel (I) nach einem der Ansprüche 1 bis 4 zur Herstellung eines Arzneimittels zur Behandlung und Prävention von Schlaganfällen, zerebralen hypoxischen Episoden und psychiatrischen Pathologien.

**10.** Verwendung einer Verbindung der Formel (I) nach einem der Ansprüche 1 bis 4 zur Herstellung eines Arzneimittels zur Prävention von durch Entzug von Tabak, Alkohol oder verschiedenen abhängigmachenden Substanzen verursachten Symptomen.

**11.** Verwendung einer Verbindung der Formel (I) nach einem der Ansprüche 1 bis 4 zur Herstellung eines Arzneimittels zur Behandlung von Schmerzen.

**12.** Verwendung einer Verbindung der Formel (I) nach einem der Ansprüche 1 bis 4 zur Herstellung eines Arzneimittels zur Behandlung von Ischämie der unteren Gliedmaßen, Arteriitis obliterans der unteren Gliedmaßen, Herzischämie, Myokardinfarkt, Herzinsuffizienz, Hautvernarbungsdefizit bei Diabetespatienten und Ulcus varicosum bei Veneninsuffizienz.

**13.** Verwendung einer Verbindung der Formel (I) nach einem der Ansprüche 1 bis 4 zur Herstellung eines Arzneimittels zur Behandlung von Entzündungsprozessen.

**14.** Verbindung der Formel (I) nach einem der Ansprüche 1 bis 4 zur Herstellung eines Arzneimittels zur Behandlung und Prävention von kognitiven Störungen; Aufmerksamkeitsstörungen; Störungen der exekutiven Funktionen in Verbindung mit Alzheimer-Krankheit, pathologischer oder normaler Alterung, Parkinson-Syndrom, Trisomie 21, psychiatrischen Pathologien, Korsakoff-Alkoholsyndrom, vaskulären Demenzen oder Schädeltraumen; motorischen Störungen, die bei Parkinson-Krankheit oder anderen neurologischen Erkrankungen beobachtet werden, oder anatomisch-histopathologischen Störungen in Verbindung mit den oben aufgeführten neurodegenerativen Erkrankungen.

**15.** Verbindung der Formel (I) nach einem der Ansprüche 1 bis 4 zur Herstellung eines Arzneimittels zur Behandlung und Prävention von Schlaganfällen, zerebralen hypoxischen Episoden und psychiatrischen Pathologien.

**16.** Verbindung der Formel (I) nach einem der Ansprüche 1 bis 4 zur Herstellung eines Arzneimittels zur Prävention von durch Entzug von Tabak, Alkohol oder verschiedenen abhängigmachenden Substanzen verursachten Symptomen.

**17.** Verbindung der Formel (I) nach einem der Ansprüche 1 bis 4 zur Herstellung eines Arzneimittels zur Behandlung von Schmerzen.

**18.** Verbindung der Formel (I) nach einem der Ansprüche 1 bis 4 zur Herstellung eines Arzneimittels zur Behandlung von Ischämie der unteren Gliedmaßen, Arteriitis obliterans der unteren Gliedmaßen, Herzischämie, Myokardinfarkt, Herzinsuffizienz, Hautvernarbungsdefizit bei Diabetespatienten und Ulcus varicosum bei Veneninsuffizienz.

**19.** Verbindung der Formel (I) nach einem der Ansprüche 1 bis 4 zur Herstellung eines Arzneimittels zur Behandlung von Entzündungsprozessen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **Green et al.** Protective Groups in Organic Synthesis. John Wiley & Sons, Inc, 1991
- **J. March.** Advances in Organic Chemistry. Wiley Interscience, 1985, 310-316
- *J. Med. Chem.,* 1992, 2392
- **Anderson ; Americ.** *Eur. J. Pharmacol.,* 1994, vol. 253, 261
- **Hall.** *Brain Res.,* 1993, vol. 600, 127
- **Houghtling.** *Mol. Pharmacol.,* 1995, vol. 48, 280